# EUROPEAN PATENT SPECIFICATION

(11) **EP 4 194 023 B1**
(45) Date of publication and mention of the grant of the patent: **03.06.2026**
(21) Application number: 21213423.3
(22) Date of filing: 09.12.2021
(51) Int. Cl.: A61M 1/16, A61M 1/34, A61M 5/14, A61M 5/36, A61M 39/24, A61M 39/28

(54) **DISPOSABLE SET AND EXTRACORPOREAL BLOOD TREATMENT APPARATUS FOR PREVENTING DEGASSING IN AN INFUSION FLUID LINE**
WEGWERFBARER SATZ UND EXTRAKORPORALE BLUTBEHANDLUNGSVORRICHTUNG ZUR VERHINDERUNG DER ENTGASUNG IN EINER INFUSIONSFLUIDLEITUNG
ENSEMBLE JETABLE ET APPAREIL DE TRAITEMENT SANGUIN EXTRACORPOREL POUR EMPÊCHER LE DÉGAZAGE DANS UNE LIGNE DE FLUIDE DE PERFUSION

(43) Date of publication of application: 14.06.2023
(73) Proprietor: Gambro Lundia AB, 226 43 Lund (SE)
(72) Inventor: POUCHOULIN, Dominique, 01390 Tramoyes (FR); FRUGIER, Alain, 69380 Chazay d'Azergues (FR)
(74) Representative: PGA S.p.A., Milano, Succursale di Lugano

(56) References cited:
- EP-A1- 3 838 306
- EP-B1- 2 192 937
- WO-A2-01/91829
- EA-B1- 033 951
- US-A1- 2017 340 796
- US-A1- 2020 054 816
- US-A1- 2020 179 588
- US-A1- 2021 213 189
- US-B2- 9 089 653

## Description

### FIELD OF THE INVENTION

The present invention relates to a disposable set and to an extracorporeal blood treatment apparatus for preventing degassing in an infusion fluid to be infused in an extracorporeal blood circuit. The present invention particularly relates to extracorporeal blood treatments, such as dialysis, blood oxygenation/CO₂ removal treatments, therapeutic plasma exchange and/or hemoperfusion, wherein a fluid is infused in the extracorporeal blood circuit at a junction point where negative pressure (pressure below external pressure, e.g. atmospheric pressure) occurs.

### BACKGROUND

The kidneys fulfil many functions, including the removal of water, the excretion of catabolites (or waste from the metabolism, for example urea and creatinine), the regulation of the concentration of the electrolytes in the blood (e.g. sodium, potassium, magnesium, calcium, bicarbonates, phosphates, chlorides) and the regulation of the acid/base equilibrium within the body, which is obtained in particular by the removal of weak acids and by the production of ammonium salts. In individuals who have lost the use of their kidneys, since these excretion and regulation mechanisms no longer work, the body accumulates water and waste from the metabolism and exhibits an excess of metabolites. In order to overcome renal dysfunction, resort is conventionally made to a blood treatment involving extracorporeal circulation within a blood circuit through an exchanger having a semipermeable membrane (dialyzer) in which the patient's blood is circulated on one side of the membrane and a dialysis liquid, comprising the main electrolytes of the blood in concentrations close to those in the blood of a healthy subject, is circulated on the other side. The patient is connected to the extracorporeal blood circuit through a withdrawal (or arterial) and a return (or venous) line, the latter having respective needles at the end portions.

A pressure difference is created between the two compartments of the dialyzer which are delimited by the semipermeable membrane, so that a fraction of the plasma fluid passes by ultrafiltration through the membrane into the compartment containing the dialysis liquid. The blood treatment which takes place in a dialyzer as regards waste from the metabolism and electrolytes results from two mechanisms of molecular transport through the membrane between the blood and the dialysis fluid.

The dialysis fluid is prepared upstream of the dialyzer by mixing pure water with a plurality of predetermined substances, such as electrolytes and buffer, to be exchanged within the dialyzer with the patient's blood. Water comes from an online port receiving purified and de-ionized tap water (e.g., by reverse osmosis), to be subsequently further filtered within the apparatus so that a substantially endless water source is provided to the blood treatment apparatus for subsequent mixing with concentrates. Alternatively, the dialysis fluid is pre-prepared and housed in respective bags to feed the dialysis apparatus, i.e. in case of acute dialysis treatments.

Treatment apparatuses configured to deliver hemofiltration or hemodiafiltration treatments or blood oxygenation/CO₂ removal treatments generally comprise an infusion line connected to the blood circuit of the disposable set: the infusion line may be used for infusing replacement fluid into the patient extracorporeal blood and/or to infuse one or more specific substances to control other blood parameters. For example, a bicarbonate solution may be infused into the blood circuit for controlling blood acid-base balance of the patient during the dialysis treatment: a bag containing a bicarbonate liquid solution is usually provided to be connected to the blood circuit in order to allow a controlled infusion. Furthermore, an anticoagulant solution, such as citrate, may be infused in the blood circuit in order to avoid blood coagulation: a bag containing a citrate or other regional anticoagulant is usually provided to be connected to the blood circuit in order to allow a controlled infusion.

Currently, the infusion of certain substances is provided upstream the blood pump on the withdrawal blood line. For example, a citrate solution is usually infused upstream the blood pump to achieve the local anticoagulation effects as soon as possible in the blood circuit. During a dialysis treatment, the blood pump generates a blood flow in the blood circuit; for example, a peristaltic pump conveys fluid by alternately squeezing and releasing the flexible tube of the pump, thereby moving the blood along an advancement direction. A pressure regimen is defined inside the blood circuit, wherein (usually) a negative pressure occurs upstream the blood pump and a positive pressure occurs downstream the blood pump. Of course, the circuit pressures in the blood lines are the consequences of several factors including (but not limited to) the blood pump pumping the fluid, the circuit pressure drops and pressure conditions at the line ends. Blood is withdrawn from a patient access through the withdrawal line and the blood pump generates the blood flow that (usually) contributes to determine a positive pressure regimen, namely a prevalence, downstream the pump, and a negative pressure regimen upstream the pump. The negative pressure, namely a pressure lower than the atmospheric pressure, sometimes occurs at the pre-blood pump (PBP) infusion line that has an injection point into the blood withdrawal line upstream the blood pump. Such negative pressure may cause or promote generation of gases, i.e. bubbles, previously dissolved in the fluid. Solutions for PBP (Pre Blood Pump) infusion, more particularly (but not exclusively) those containing bicarbonate, delivered e.g., during CRRT therapies, degas in case they are infused in negative pressure conditions. Some degassing may also be visually observed in the blood set along the PBP infusion line, from the outlet of the PBP pump to the PBP-access junction connector. Degassing of solutions along the PBP circuit, from the outlet of the PBP pump to the PBP solution injection site, is at the origin of numerous complaints dealing with both simple presence of air bubbles in the circuit (e.g., suspicion of leak) and/or accumulation of air and/or foam in the deaeration chamber requesting regular monitoring and adjustments.

Notably, the infusion fluids may lead very easily to gas generation, alias degassing, when exposed to the low pressure occurring upstream the blood pump: a degassing chamber arranged downstream the blood pump may be provided to trap the air bubbles, thereby reducing the health risks for the patient: anyhow, the degassing chamber is ineffective to prevent the cause of the fluid degassing process. In addition, the more is the amount of bubbles in the blood circuit, the more is the time frequency requested to periodically service the degassing chamber. Accumulation of foam in the deaeration chamber may lead to various failures when in excessive amounts (including loss of circuit and failure to return blood). Further, when assuming development of some blood activation at blood-air interface, a clotting risk increases.

Document EP2192937B1 discloses an infusion apparatus and a control method for an infusion apparatus for infusing a medical fluid to a patient via an extracorporeal blood circuit.

Document US20170340796A1 discloses an addition line for adding an infusion solution to a fluid, which flows in an extracorporeal blood circuit, and wherein the addition line comprises a pressure release valve or a check valve.

Document US9089653B2 discloses an extracorporeal blood treatment system having reversible blood pumps and infusion lines comprising check valves.

### OBJECTIVE OF THE INVENTION

The objective of this invention is therefore to at least partially solve one or more of the drawbacks described above.

A first objective is to provide a disposable set and an apparatus for reducing or preventing degassing in an infusion fluid when the latter is infused in a blood line upstream the blood pump in an extracorporeal blood treatment apparatus.

A further aim is to increase safety level for the patient health, reducing risk of embolism and/or blood clotting.

A further objective is to reduce the workload of the medical personnel in servicing the degassing chamber, in order to reduce required interventions.

An aim is to increase the reliability of automatic level management in the chamber and further to decrease field problems and complaints related to degassing of solutions.

A further objective is to provide a device for preventing degassing in an infusion fluid and, at the same time, keeping manufacturing costs of the device low.

At least one of the above objectives is achieved by the present invention. These objects and more, which will appear more from the following description, are substantially achieved by a disposable set and an apparatus in accordance with one or more of the following claims and/or aspects.

A disposable set according to the invention is disclosed in the appended claims 1-10

An extracorporeal blood treatment apparatus according to the invention is disclosed in the appended claims 11-13**.**

### DRAWINGS

Some embodiments and some aspects of the invention will be described below with reference to the attached drawings, provided for illustrative purposes only, wherein:
- Figures 1 and 2 are schematic views of possible different embodiments of an extracorporeal blood treatment apparatus comprising the disposable set according to the present invention;
- Figure 3 is a section view of a fluid connector housing a one-way valve according to an embodiment of the disposable set of the present invention;
- Figures 4-6 are a schematic views of a flow passage restrictor according to different embodiments of the disposable set;
- Figure 7 is a schematic view of a disposable set according to the present invention fluidly connected to a (schematic) ECMO circuit.

### DEFINITIONS

In this detailed description, corresponding parts illustrated in the various figures are indicated with the same numerical references. The figures may illustrate the invention through non-scale representations; therefore, parts and components illustrated in the figures relating to the object of the invention may relate exclusively to schematic representations.

The terms *"upstream"* and *"downstream"* refer to a direction or trajectory of advancement of a fluid configured to flow within the connector or along the fluid line or duct during normal usage of the apparatus, for example during an extracorporeal blood treatment. During normal use of the apparatus the blood pump pumps blood from the patient vascular access along the blood withdrawal line, cross the filtration unit and back to the patient along the blood return line. Infusion fluids are infused from the respective fluid sources towards the blood circuit and into the blood. Dialysis fluid (if any) flows from the dialysis line to the filtration unit and towards the effluent line. Blood flow and dialysis flow are countercurrent in the filtration unit.

In an ECMO circuit, the blood direction is defined by the respective blood pump from the blood withdrawal duct towards the blood return duct.

We define the *"dialysis fluid"* as the treatment fluid introduced to the second chamber of the filtration unit 2. The dialysis fluid may be on-line prepared or pre-packaged in sterile bags. Usually in CRRT apparatuses/applications the dialysis fluid, but also the replacement fluids (possibly also regional anticoagulant fluid and/or ion re-establishing solution fluid) are contained in (disposable) bags.

We define the *"dialysate"* or *"effluent"* as the fluid from the outlet from the second chamber of the filtration unit 2. Dialysate or effluent is the spent dialysis fluid, comprising the uremic toxins removed from the blood and may include ultrafiltrate fluid.

We define *"regional anticoagulant"* as a substance which, once mixed with extracorporeal blood, substantially prevents blood coagulation in the extracorporeal blood circuit and which is quickly metabolized by the patient, thus avoiding systemic anticoagulation.

We define the term *"degassing"* as a process wherein gases dissolved in a fluid, such as an infusion fluid or blood, tend to get free due to a local low pressure or due to fluid warming, which leads to separation of the gases from the liquid phase of the fluid, consequently generating bubbles into the fluid.

We define *"negative pressure"* a pressure below the local atmospheric pressure. We define *"positive pressure"* a pressure above the local atmospheric pressure.

### DETAILED DESCRIPTION

### Disposable set 100

Reference number 100 is directed to a disposable set for an extracorporeal blood treatment apparatus, such as a hemodialysis apparatus for performing a haemodialysis treatment (HD), an ultrafiltration apparatus for performing an ultrafiltration treatment (UF), a haemofiltration apparatus for performing a haemofiltration treatment (HF) or a haemodiafiltration apparatus for performing a haemodiafiltration treatment (HDF).

Alternatively, the disposable set 100 may be directed to perform an extracorporeal blood treatment such as a Therapeutic Plasma Exchange (TPE) treatment. The TPE treatment is a procedure wherein the patient's blood passes through an apheresis machine for plasma filtration and removal: plasma is then replaced by a replacement fluid, such as a plasma from a donor, albumin, or saline.

Alternatively, the disposable set 100 may be directed to perform an extracorporeal blood treatment such as a HemoPerfusion treatment for blood purification: in particular HemoPerfusion treatment consists of the passage of the patient's blood through a device, usually a column, which contains adsorbent particles configured to remove toxins from blood, i.e. in case of treatment of poisoning.

Alternatively, the disposable set 100 may be directed to perform an extracorporeal blood treatment for CO₂ removal treatment from the blood: the CO₂ removal treatment may be performed during a dialysis treatment, or may be performed by itself through a blood circuit ad hoc.

Furthermore, the disposable set 100 may be connected to a further extracorporeal blood treatment apparatus, such as an apparatus for blood oxygenation, namely an ExtraCorporeal Membrane Oxygenation ECMO treatment apparatus.

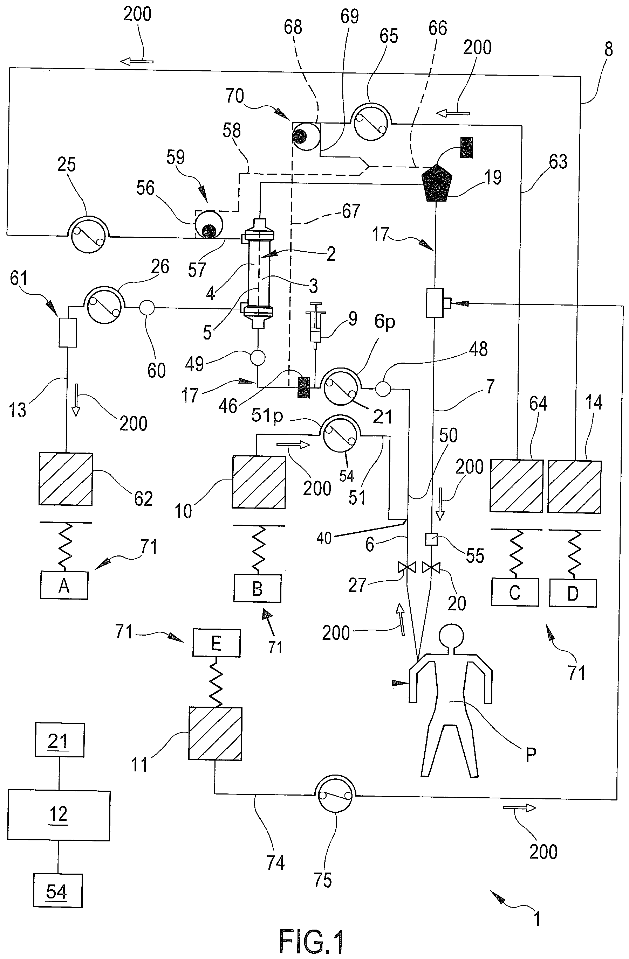

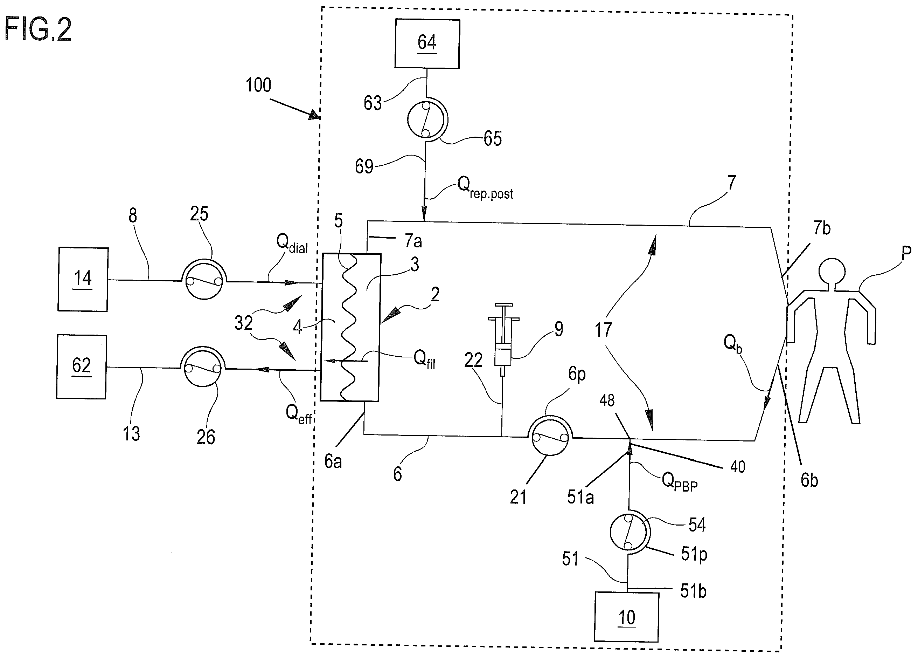

According to the medical fields listed above, the disposable set 100, schematically shown in figures 1 and 2, comprises blood and fluid lines configured to be associated to the respective treatment apparatus 1, for example to peristaltic pumps to promote fluid flow and respective sensors and actuators to operate the circuit. Here after a detailed description of the disposable set is provided. In this regard, the pumps are not part of the disposable set 100, even if for sake of simplicity, the dot lines in figure 2 encompass also pumps (e.g., infusion pump 54, blood pump 21 and post-infusion pump). Syringe pump 9 and bags 10 and 64 may be part or may be not part of the disposable set depending on its configuration.

The disposable set 100 comprises at least one filtration unit 2, which is configured to treat the blood withdrawn from the patient. The filtration unit 2 may be a filter for performing one between a haemodialysis treatment (HD), an ultrafiltration treatment (UF), a haemofiltration treatment (HF) and a haemodiafiltration treatment (HDF). The filtration unit 2 may alternatively be an absorber unit, or sorbent cartridge for a sorbent system in case of hemoperfusion treatments.

In an embodiment, the filtration unit 2 has a primary chamber 3 and a secondary chamber 4 separated by a semi-permeable membrane 5, wherein the primary chamber 3 receives the blood withdrawn from the patient, while the secondary chamber 4 receives waste products and fluid removed from the blood and discharges it through an outlet connected to an effluent fluid line 13. Depending upon the treatment, the membrane of the filtration unit may be selected to have different properties and performances. According to a further embodiment, the secondary chamber of the filtration unit 2 further comprises, in addition to the outlet, an inlet configured to receive fluid, i.e. dialysis fluid, from a dialysis liquid supply line 8.

The disposable set may comprise the effluent fluid line 13 connected to the outlet of the dialyzer 2: the effluent fluid line 13 may also comprise a respective effluent pump tract configured to be engaged to a dialysate pump 26.

Analogously, in the case wherein the dialyzer comprises an inlet, the disposable set may comprise a supply line 8 connected to the inlet of the dialyzer and configured to deliver dialysis fluid to the dialyzer 2. The dialysis liquid supply line 8 also comprises a respective supply pump tract configured to be engaged to a dialysis fluid pump 25 of the extracorporeal blood treatment apparatus.

The blood circuit further comprises a blood withdrawal line 6 extending between a first end 6a connected to the filtration unit 2 and a second end for connection to a patient P. In case the filtration unit 2 comprises the primary and secondary chambers, the blood withdrawal line 6 extends between a first end 6a connected to the inlet of the primary chamber 3 of the filtration unit 2 and a second end 6b for connection to a patient P. The blood withdrawal line 6 is configured to receive blood from the patient P and carry the blood along a withdrawn direction 200 from the second end 6b to the first end 6a of the blood withdrawal line 6.

The blood circuit 17 further comprises a blood return line 7 extending between a first end 7a connected to the filtration unit 2 and a second end 7b for connection to said patient P. In case the filtration unit 2 comprises the primary and secondary chambers, the blood return line 7 extends between a first end 7a connected to the outlet of the primary chamber 3 of the filtration unit 2 and a second end 7b for connection to a patient P. The blood return line 7 is configured to receive blood from the outlet of the filtration unit 2 and carry the blood along a return direction defined from the first end to the second end of the blood return line 7.

The withdrawal line 6 and the return line 7 may be connected to the blood stream of the patient through a vascular access, through a needle, a catheter, or an access device.

The withdrawal line 6 and the return line 7 may be made of a flexible material, for example PVC or other plastic based bio compatible material: the blood lines 6, 7 may also be transparent to allow an operator to see the blood flowing within the lines.

The blood withdrawal line 6 of the blood circuit may have a fluid passage section comprised between 3 mm² and 20 mm² corresponding to inner diameters between 2 mm and 5 mm (the more common inner diameters are included between 2.5 mm and 4.5 mm).

The blood withdrawal line 6 includes a pump tract 6p configured to be engaged to a blood pump 21 of the extracorporeal blood treatment apparatus which is configured to generate a blood flow and the blood circulates in the blood circuit in a direction 200 from the blood withdrawal line 6 towards the filtration unit 2. At least during an operating condition of the apparatus, usually a positive pressure regimen is experienced downstream the blood pump tract 6p, and an access negative pressure regimen is experienced upstream the blood pump tract 6p. Notably the access negative pressure is lower than the atmospheric pressure.

In one example, the blood-pump 21 may be implemented by a pump-rotor element integrated with the dialyzer and operably connected to a magnetic field for its operation.

The blood pump tract 6p may be a portion of the blood withdrawal line 6 itself, which is interposed between the first end 6a and the second end 6b of the blood withdrawal line 6.

Thus, the blood withdrawal line 6 comprises:
- the blood pump tract 6p extending between the first end 6a and the second end 6b,
- a first tract extending between the first end 6a and the blood pump tract 6p,
- a second tract extending between the second end 6b of the blood withdrawal line 6 and the blood pump tract 6p.

The blood pump tract 6p may be different with respect to the first and the second tracts of the blood withdrawal line 6 in terms of dimension and/or material. In particular the blood pump tract 6p may have an external dimension, i.e. an external diameter, bigger than an external dimension, i.e. an external diameter, of the first and/or second tracts of the blood withdrawal line. In addition or alternatively, the blood pump tract 6p may have an internal dimension, i.e. an internal diameter, defining the fluid passage of the blood pump tract 6p, which is bigger than an internal dimension, i.e. an internal diameter, of the first and/or second tracts of the blood withdrawal line. The blood pump tract 6p may be a different tube segment with respect to the first and the second tracts, wherein the blood pump tract 6p is engaged/coupled to the first and the second tracts by a gluing or welding step performed during a manufacturing process of the disposable set 100. Usually, the blood withdrawal line 6 has uniform section with the exclusion of the pump tract 6p that may have a slightly bigger inner section, e.g., 6-8 mm.

The blood pump tract 6p may also has a stiffness/elasticity different from a stiffness of the first and the second tracts of the blood withdrawal line 6: for example the blood pump tract 6p may be more flexible or elastic than the first and the second tracts of the blood withdrawal line 6. The blood pump tract 6p, in order to withstand the fatigue stresses caused by the peristaltic pump 21 of the external blood treatment apparatus, is made by a more flexible/elastic material with respect to a material of the first and/or second tracts of the blood withdrawal line 6.

The blood circuit further comprises an infusion line 51 extending between a first end 51a connected to the blood withdrawal line 6 upstream the blood pump tract 6p at a fluid access, and a second end 51b for connection to an infusion substance source: the blood pump tract 6p is interposed between the filtration unit 2 and the first end 51a of the infusion line 51.

The blood withdrawal line 6 and the infusion line 51 may be made by the same material, i.e. PVC, silicone, or other plastic based material. The blood withdrawal line 6 and the infusion line 51 may also have the same geometry, for example a circular cross section having constant internal diameter and constant external diameter. Notably, both the blood withdrawal line 6 and the infusion line 51 are flexible.

The infusion line 51 may have a fluid passage section comprised between 0,7 mm² and 20 mm² or the fluid passage section may have a diameter comprised between 1 mm and 5 mm.

The infusion substance source 10, connected or configured to be connected to the infusion line 51, may be an infusion bag. The bag may house a fluid infusion solution comprising one between bicarbonate, acetate, lactate, citrate, a replacement fluid, saline, and a regional anticoagulant solution. Notably, the disposable set may comprise the bag, which is connected to the second end 51b of the infusion line 51. The infusion line 51 may further comprise a respective infusion pump tract 51p interposed between the first and 51a the second end 51b of the infusion line 51: the infusion pump tract 51p of the infusion line 51 is configured to be engaged by an infusion pump 54, i.e. a peristaltic pump, configured to determine, at least during an operating condition of the infusion pump 54, a positive pressure downstream the infusion pump tract 51p to allow infusion fluid to flow in a direction from the second to the first end of the infusion line 51 towards the blood withdrawal line 6. The infusion pump tract 51p may comprise the same features previously described according to the blood pump tract 6p of the blood withdrawal line 6.

Thus the infusion line 51 comprises:
- the infusion pump tract 51p extending between the first end 51a and a second end 51b,
- a first tract extending between the first end of the infusion line 51 and the first end of the infusion pump 54 tract,
- a second tract extending between the second end of the infusion line 51 and the second end of the infusion pump 54 tract,
wherein the infusion pump tract is different from the first and the second tracts of the infusion line 51 in terms of dimensions and/or material. In particular the infusion pump tract may have an external dimension, i.e. an external diameter, bigger than an external dimension, i.e. an external diameter, of the first and/or second tracts of the infusion line 51. In addition or alternatively, the infusion pump tract may have an internal dimension, i.e. an internal diameter, defining the fluid passage which is bigger than an internal dimension, i.e. an internal diameter, of the first and/or second tracts of the infusion line 51. The infusion pump tract 51p may be a different tube segment with respect to the first and the second tracts, wherein the infusion pump tract is engaged/coupled to the first and the second tracts by a gluing or welding step performed during a manufacturing process of the disposable set 100.

The infusion pump tract 51p may also has a stiffness/elasticity different with respect to a stiffness of the first and the second tracts of the infusion line 51: for example the infusion pump tract 51p may be more flexible/elastic than the first and the second tracts of the infusion line 51. The infusion pump tract, in order to withstand the fatigue stresses caused by the peristaltic infusion pump 54 of the external blood treatment apparatus, is made by a more flexible/elastic material with respect to a material of the first and/or second tracts of the infusion line 51.

The blood circuit 17 comprises an intersection portion where the infusion line 51 is joined to the blood withdrawal line 6 to allow the infusion fluid, flowing within the infusion line 51, to be infused into the blood withdrawal line: in particular the intersection portion includes an infusion connector 48 having a connector body defining an internal volume.

The infusion connector comprises a blood inlet 48a and a blood outlet 48b fluidly communicating each other and with the internal volume of the connector, and wherein the blood inlet 48a is connected to the blood withdrawal line 6 and faces the second end 6b of the blood withdrawal line 6, while the blood outlet 48b is connected to the blood withdrawal line 6 and faces the blood pump tract 6p of the blood withdrawal line 6. In particular the blood flows within the infusion connector in the withdrawal direction 200 from the blood inlet 48a to the blood outlet 48b of the infusion connector 48. The infusion connector 48 further comprises an infusion inlet 48c fluidly communicating with the internal volume of the connector body and connected to the first end 51a of the infusion line 51. Thus, the infusion inlet 48c, the blood inlet 48a and the blood outlet 48b are in fluid communication each other defining a three-way connector having two inlets and one outlet.

The infusion connector 48 may be made by plastic material: in particular the infusion connector is generally stiffer than the infusion line 51 and/or of the blood withdrawal line 6. On the contrary, the infusion line 51 is flexible, in particular more flexible than the infusion connector 48. Analogously, the blood withdrawal line 6 is more flexible than the infusion connector: for example, the blood withdrawal line 6 may be as flexible as the infusion line 51.

The infusion connector 48 may be in one piece with the blood withdrawal line 6 and with the infusion line 51: in this case, the first end of the infusion line 51 is welded or glued to the infusion inlet of the infusion connector. Also the blood withdrawal line 6 is welded or glued to the blood inlet and to the blood outlet of the infusion connector 48. The term *"welded"* may refer to thermal or chemical welding between the blood or infusion line 51 and the infusion connector. Thus the infusion connector 48, the blood withdrawal line 6 and the infusion line 51 may define a non-separable fluid line set. In particular the disposable set including the blood withdrawal line 6, the infusion line 51, the blood return line 7 and the dialyzer 2, may be in one piece, defining thereby a one piece disposable set 100. In particular no removable connectors may be provided in the disposable set for connection of the blood circuit to the infusion line 51 and the filter unit 2.

Notably the infusion fluid may be for example a replacement fluid or a saline or a regional anticoagulation fluid, depending on the specific treatment and/or on the specific step of an apparatus working sequence (e.g., priming vs patient blood treatment). The infusion fluid may include a buffer (e.g., bicarbonate, acetate or lactate), one or more electrolytes (e.g., sodium, magnesium, calcium, potassium, etc.), or a regional anticoagulant, such as citrate (e.g., trisodium citrate or citric acid). According to one embodiment, the infusion line 51 is connected to the infusion connector 48 at a coupling portion defining a discontinuity-free coupling in the flow passage. In other terms, an internal flow passage in substantially constant from the infusion line 51 up to the infusion connector, including the coupling portion: de facto, the coupling portion does not define a discontinuity in the internal flow passage, in order to avoid turbulences in the fluid flow.

Notably, the negative pressure regimen in the blood line of the disposable set extends at least between the blood pump tract 6p of the blood withdrawal line 6 and the intersection portion with the infusion line 51: in particular the negative pressure regimen may also extend at least between the blood pump tract 6p of the blood withdrawal line 6 and the second end 6b of the blood withdrawal line 6 at the connection with the patient.

The disposable set 100 further comprises a pressure damper 40 arranged towards or at the first end of the infusion line 51: in particular the pressure damper 40 may be arranged at the infusion connector, namely at the intersection between the blood withdrawal line 6 and the infusion line 51, or on the infusion line 51 in proximity of the intersection, i.e. at a distance from the intersection comprised between 0.1 cm and 6 cm, more in particular between 0.2 and 3 cm. The pressure damper 40 is arranged on the infusion line 51 between the infusion pump tract 51p and the blood withdrawal line 6.

The pressure damper 40 is configured to prevent, or to reduce an amount of, the access negative pressure to extend in the infusion line 51 upstream the pressure damper 40 with respect to the direction of the infusion fluid: the direction of the infusion fluid is directed from the second end 51b to the first end 51a of the infusion line 51. As already mentioned, the access negative pressure may cause degassing especially in the infusion fluid, thereby generating bubbles, which are problematic when present in the blood circuit for all the above explained reasons. Thus, the pressure damper 40 allows the infusion fluid in the infusion line 51 to be, during an operating condition of the blood treatment apparatus, at a pressure higher than the access negative pressure, thereby reducing or preventing degassing of the infusion fluid.

In order to achieve the task of reducing or preventing the negative pressure in the infusion line, two different approaches are provided. In a first approach (not being part of the invention), a suitable restriction of the flow passage in the infusion line is obtained, but without preventing the fluid flow (i.e., the fluid passage, though restricted, is always open independent on the apparatus working condition). In a second approach a suitable one-way valve is used with an opening threshold so to prevent fluid passage unless a certain pressure differential over the one way valve is present in the infusion line.

According to the first embodiment as indicated not part of the invention, the pressure damper 40 may be a flow passage restrictor 41 which comprises a damper passage section configured to allow the infusion fluid to pass through: the damper passage section is smaller than the fluid passage section of the infusion fluid: for example the damper passage section may be at least 50% smaller than the fluid passage section, in particular at least 80% smaller, in particular more than 90% smaller. The flow restrictor may define a fluid passage section having circular shape.

The flow passage restrictor 41 of the pressure damper 40 may comprise a septum, e.g., having circular shape, having a fluid passage which reduces the fluid passage section with respect to the passage section of the infusion line 51. In order to achieve a sufficient pressure drop considering a typical infusion rate of about 1000 ml/h, the septum should define a fluid passage having a cylindrical section with a diameter less than 0.4 mm in case a fluid passage length lower than 60 mm is desired. Though it may be difficult to mold a plastic element with an inner diameter of 0.5 mm or less, a metallic portion may be eventually used. Needles such as 33G (0.24 mm), 32G (0.26 mm), 30G (0.3 mm), 27G (0.41 mm) or 26G (0.45 mm) may be used. Portions of the required length may be indeed obtained.

The fluid passage axially extends along a flow direction of the infusion line 51 by a fluid passage length comprised between 1 mm and 30 mm, in particular between 2 mm and 15 mm, in particular between 4 mm and 10 mm. According to the embodiment comprising the septum, the flow passage restrictor 41 of the pressure damper 40 may define a sharp discontinuity for the fluid flow in the infusion line 51. According to an embodiment, the damper passage section of the flow passage restrictor 41 is substantially constant along the fluid passage length. Notably, the flow passage restrictor 41 defines an aperture, which puts the infusion line 51 in fluid communication with the blood withdrawal line 6. The aperture is fixed in size: de facto the latter cannot be modified by an operator during the treatment. According to an embodiment, the flow restrictor of the pressure damper 40 may be arranged into the infusion connector, in particular inside the infusion inlet 48c of the infusion connector 48.

Alternatively, the flow restrictor of the pressure damper 40 may be arranged on the infusion line 51 upstream, with respect to the flow direction of the infusion fluid, the infusion connector 48 and as close as possible to the blood withdrawn line 6. For example, the pressure damper 40 may be a damper connector interposed between an upstream and a downstream tract of the infusion line 51, wherein the flow restrictor is arranged within said damper connector: this damper connector may extends along the infusion fluid direction by a length comprised between 5 mm and 30 mm, in particular between 10 and 20 mm. According to this embodiment, the damper connector may be stiffer than the infusion line 51 and made of a material different and stiffer than the material of the infusion line 51, for example the damper connector may be made of metal and the infusion line 51 of flexible PVC or silicone. Notably, when a passage restrictor is provided, the infusion line 51 defines an internal flow passage having a fluid passage section substantially constant up to the passage restrictor of the pressure damper 40: thus the passage restrictor defines a discontinuity in the fluid passage section to prevent the negative pressure to extend into the infusion line 51.

Indeed, the flow passage restrictor 41 is configured to define a pressure drop in the infusion fluid during an operating condition of the treatment apparatus, in particular when an infusion fluid flows within the infusion line 51 towards the blood withdrawal line 6. The pressure drop is considered as a differential pressure between the infusion line and the blood withdrawal line upstream the blood pump tract: in particular the pressure drop is considered as a differential pressure between a section just upstream the pressure damper 40, and a section just downstream the pressure damper 40.

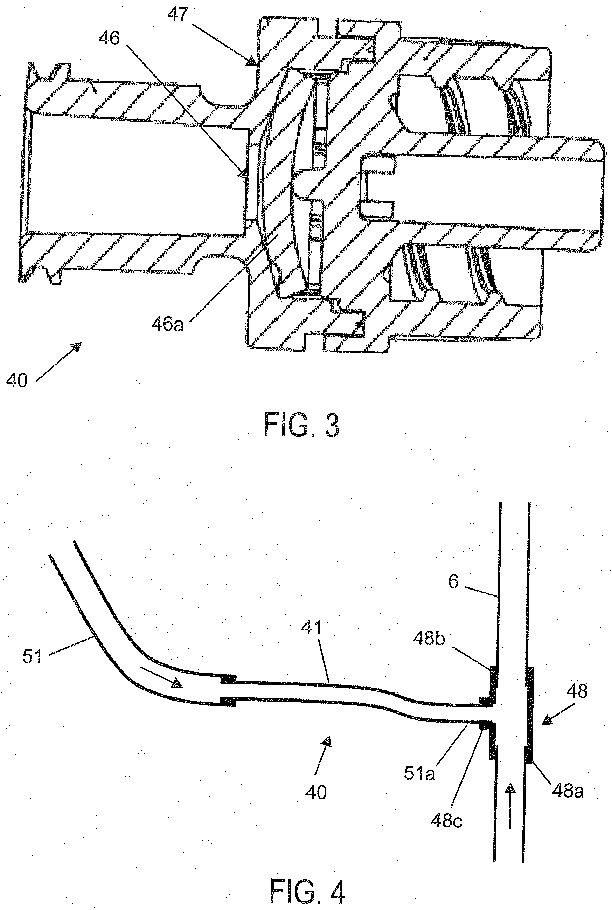

Figure 4 shows a further embodiment not part of the invention wherein the flow passage restrictor 41 comprises a tube tract defining the first end of the infusion line: the tube tract has an internal diameter lower than the internal diameter of the infusion line and extends by a length higher than the passage restrictors described in the preceding embodiments. In particular the damper passage of the tube tract extends by a length comprised between 30 mm and 200 mm, in particular between 40 mm and 160 mm, more in particular between 55 mm and 150 mm, and the damper passage section extends by a diameter comprised between 0.3 mm and 0.6 mm, in particular between 0.35 mm and 0.55 mm.

More in detail, and according to a specific embodiment, if the damper passage section has a diameter substantially equal to 0.5 mm, the damper passage extends by a length substantially equal to 145 mm optionally ± 15 mm, while if the damper passage section has a diameter substantially equal to 0.4 mm, the damper passage extends by a length substantially equal to 60 mm optionally ± 10 mm. The diameter of the damper passage is substantially constant over the length.

Notably the tube tract defining the flow passage restrictor 41 may be made of the same material of the rest of the infusion line, i.e. a flexible material, such as medical grade PVC, silicone, or of another bio compatible material.

In order to avoid degassing of the infusion fluid, the internal diameter of the flow passage restrictor 41 depends on the length of the flow passage restrictor 41 and vice versa: indeed, given the properties of the infusion fluid and the flow rate set for the infusion fluid, the pressure drop across the pressure damper is defined by the damper passage section and by the length of flow passage restrictor 41. In particular the damper passage section is proportional to the length of the flow passage restrictor 41: in other terms, the smaller is the damper passage section, the lower is the length of the flow passage restrictor 41 needed to obtain a sufficient pressure drop to avoid degassing of the infusion fluid in the infusion line 51.

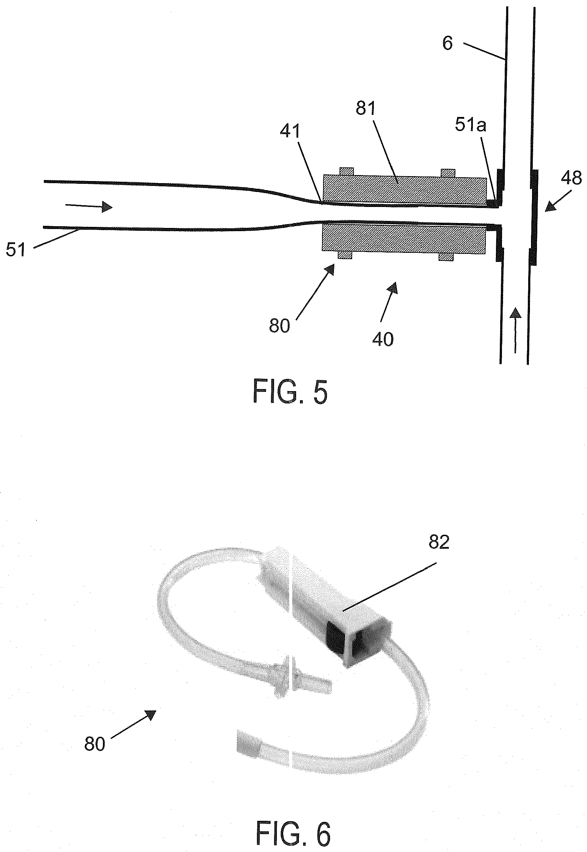

Still according to a further embodiment not part of the invention, the flow passage restrictor 41 may be defined by a clamp 80 configured to clamp the infusion line 51 and to deform the infusion line so that the internal lumen is reduced (but not completely closed as normal clamps do). Thus the clamp acts on the external surface the infusion line 51, radially compressing the line and reducing the internal damper passage section. The clamp 80 may comprise a portable clamp 81, as schematically shown in figure 5, comprising a clamping seat configured to receive a portion of the infusion line 51 and having a fixed size to determine a predefined lumen reduction of the infusion line 51 along a certain tube length: this type of clamp is designed according to a specific geometry, i.e. the size, of the infusion line, in order to cause a predefined lumen restriction. Also in this embodiment, a relation exists between the lumen restriction and the length of the tube portion that is restricted. The longer the tube portion that is restricted, the lower is the lumen restriction and vice versa.

Alternatively, the clamp 80 may comprise a variable clamp 82, as shown in figure 6, comprising a clamping seat configured to receive a portion of the infusion line 51 and having a regulator for varying the lumen reduction of the infusion line 51.

Notably, the flow passage restrictor 41 defined by the clamp 80 is defined by the same geometrical features already described according to the previous embodiment, in particular in terms of damper passage section of the flow passage restrictor 41 and the length thereof. In other terms, the clamp has a predefined length and is designed to determine the damper passage section of the flow passage restrictor 41 in order to reduce or avoid degassing of the infusion fluid.

According to a different embodiment of the present invention, shown in figure 3, the pressure damper 40 may comprises a one-way valve 46 configured to allow fluid passage only along the infusion direction directed from the infusion line 51 towards the blood withdrawal line. The one-way valve 46 is configured to move between an open position, in which the fluid passage is allowed in the infusion direction, and a closed position, in which fluid passage is prevented in the infusion direction, in particular in both directions. Notably, the one-way valve only allows fluid flow only in one direction, namely the infusion direction: fluid flow in the opposite direction is always prevented.

The one-way valve 46 is preset at an opening pressure threshold to switch between the open and the closed position and vice versa, so that if a differential pressure between an upstream section and a downstream section of the one-way valve 46 is equal or higher than this opening pressure threshold, the one-way valve 46 is configured to switch to or to maintain the open position. Alternatively, if the differential pressure is lower than this opening pressure threshold, the one-way valve 46 is configured to switch to or to maintain the closed position. Notably, the opening pressure is defined as the differential pressures just upstream and just downstream section the one-way valve: in other terms, the upstream and downstream pressures al local pressure around the internal membrane of the one-way valve.

Notably, the differential pressure is defined between a high pressure at the upstream section of the one-way valve 46, and a low pressure at the downstream section of the one-way valve 46: the high pressure is higher than the low pressure. In particular the high pressure is the pressure in the infusion line 51 upstream, with respect to the infusion direction, to the one-way valve, while the low pressure is the access negative pressure proximal to the catheter/vascular access. The preset opening pressure threshold of the one-way valve 46 corresponds to a differential pressure value comprised between 160 and 500 mmHg, in particular between 190 and 450 mmHg, more in particular between 200 and 400 mmHg, more in detail between 240 and 350 mmHg. These pressure ranges correspond to pressure regimens that normally raise when usual blood flow rates and infusion flow rates are generated within the disposable set. It is clear that the one-way valve opening threshold may be configured to the most common working conditions of the apparatus and, as above discusses, may be optimal for each and every working condition of the apparatus.

The one-way valve 46 may comprise an internal diaphragm 46a movable between the open position and the closed position: the internal diaphragm may be a flexible membrane made of a material between silicone, rubber, PVC or the like. The internal diaphragm is preloaded in the closed position to define the preset opening pressure threshold. In particular the internal diaphragm may have a semispherical shape so that a central portion of the internal diaphragm is axially shifted with respect to the external contour. The geometrical features of the internal diaphragm and the material thereof define the preload, and therefore the preset opening pressure threshold.

According to an embodiment, the disposable set comprises a Luer lock connector 47 housing the one-way valve 46, wherein the internal diaphragm is within an internal fluid passage of the Luer lock connector. The Luer lock connector may be arranged on the infusion line 51, i.e. towards the first end 51a of the infusion line 51, for example at a distance from the first end of the infusion line 51 comprised between 0.1 cm and 6 cm, more in particular between 0.2 and 3 cm.

Alternatively, the one-way valve 46 may be arranged inside the infusion connector 48, in particular inside the infusion inlet 48c of the infusion connector 48.

### Extracorporeal blood treatment apparatus 1

With reference to figure 1, the numeral 1 globally refers to the extracorporeal blood treatment apparatus, in particular for intensive care therapies, configured to receive the disposable set previously described. The extracorporeal blood treatment apparatus 1 is designed for performing any one of treatments like e.g., hemodialysis, hemofiltration, hemodiafiltration, and ultrafiltration.

The apparatus according to figure 1 is particularly designed for continuous renal replacement therapies (CRRT). CRRT systems are configured for delivering very specific treatments designed for patients versing in acute states of illness and who have temporarily lost their kidney function in its entirety. In this respect, CRRT systems may be structurally and/or operationally different from extracorporeal blood treatment systems designed for chronic patient care. In contrast to chronic patients, acute patients temporarily experience complete loss of their kidney function typically due to a contemporaneous state of severe injury or during recovery from surgery. Consequently, acute patients are often extremely weak and typically not in a condition to be submitted to regular dialysis treatment, which may further deteriorate their state and lead to serious and possibly life-threatening complications. Under circumstances as described, CRRT systems are designed to individually treat a patient exhibiting very poor health, without inducing further stress to the patient body, in particular without allowing vital parameters pertaining to the patient's blood to deviate from ideal or near-ideal values. Within the scope of this document CRRT systems are, thus, inherently characterized by one or more of the following features. CRRT involves renal replacement therapy, meaning an adjuvant therapy aimed firstly at facilitating continuous fluid removal in diuretic-resistant or acute renal failure patients. Therefore, CRRT systems inherently require a continuous net fluid removal from the patient. In other words, a CRRT system requires a fluid balance control system, such as a weight loss control system, configured to generate a continuous net weight loss rate (as opposed to merely controlling parameters to enable achieving a desired target weight loss as typically found in chronic patient care). Furthermore, acute patients experience extravascular fluid overload, which cannot be safely removed within a short period of time (e.g. within a few hours of chronic treatment) without causing potentially severe consequences (e.g. hypovolemic shock, arrhythmia, hypoxemia, hypoventilation, etc.). Therefore, a CRRT system must inherently include a much more accurate control over system parameters, in particular flow rates, in order to ensure that the required low flow rates of both blood circulating extra-corporeally and of treatment fluid (infused in the extracorporeal circuit or diffused through the dialyzer) are used. Moreover, CRRT treatment is performed continuously (e.g. for days or even weeks, without interruption/with minimal interruptions e.g., downtimes to change bags). Therefore, treatment settings in CRRT are based on flow rate settings, rather than settings pertaining to some specified treatment time (which would be unknown as acute patients may require treatment for an unknown time). Consequently, operation of CRRT systems cannot be based on some pre-defined absolute weight loss to be achieved, but rather on a meticulously controlled fluid balance in the patient, requiring continuous adjustments to a number of operating parameters, which have to be controlled and maintained during the entire (and a priori unknown) treatment time, based on a set weight-loss rate. Additionally, CRRT renal replacement therapy involves therapy substituting kidney functions for a relatively long time period and, thus, a CRRT system further requires at least either fresh dialysis liquid exchange in the dialyzer (in order to remove unwanted substances from blood and to add desired substances to the blood by diffusion) and/or fresh infusion fluid in combination with ultrafiltration (in order to remove unwanted substances from blood and to add desired substances to the blood by convection).

At least for the reasons set forth above, CRRT systems need to exhibit specific technical features enabling the system to:
- allow setting of a weight loss rate,
- continuously remove excess water in accordance with a set weight loss rate,
- operate continuously at comparably low flow rates compatible with CRRT, and
- Balance ion equilibrium by means of proper dialysis being performed and/or by means of substitution fluid continuously being delivered at controlled flow rates.

Finally, in order to set up a CRRT apparatus as soon as possible, the CRRT machine is dressed using an integrated disposable set 100, wherein all the lines and the filtration unit are grouped together and already properly connected in the disposable set. Further, all the fluids are contained in pre-packaged bags (dialysis fluid or replacement fluids in bags of e.g., 2, 5 or 10 litres each) or pre-packaged syringes (heparin and/or concentrated calcium replacement solution).

The apparatus 1 of figure 1 has an extracorporeal blood circuit 17, which takes blood from a patient P, for instance through a needle or a catheter or an implanted port or other access device (not shown), introduced into a vein or artery of said patient, and through the blood withdrawal line 6 takes said blood, for instance continuously, to the filtration unit 2.

The blood passes through the primary chamber 3 of the filtration unit 2 and, through the blood return line 7, the treated blood is carried back to the patient. In the example of figure 1, the connection with the infusion line 51 is provided immediately downstream from the blood collecting zone on the blood withdrawal line 6. In particular, the machine is equipped with the infusion substance source 10, containing an infusion fluid comprising for example one between bicarbonate, citrate or citric acid a replacement fluid, saline, and a regional anticoagulant solution; by using the infusion pump 54, for instance a peristaltic pump, it is possible to control the fluid flow within said infusion line 51 by introducing the infusion fluid directly into the blood with a direct connection to the blood withdrawal line 6. After defining a direction of fluid (blood) circulation 200 (during normal use of the apparatus) from the blood withdrawal line 6 towards the filtration unit 2 and from the latter through the blood return line 7 towards the patient P, a known blood pressure sensor 48, which shall not be described in further detail, may be arranged immediately downstream the infusion line 51, namely between the infusion line 51 and the blood pump 21. The apparatus comprises the blood pump 21 for controlling and managing the suitable blood flow Q_{b} in the circuit. The blood pump 21 is generally a peristaltic pump acting either on the blood withdrawal line (as shown e.g. in figure 1) and/or on the blood return line. An operator may enter a set value for the blood flow rate Q_{b} through a user interface 15 and a control unit 12, during treatment, is configured to control the blood pump based on the set blood flow rate. Note that, alternatively, the blood pump 21 may be automatically controlled with no need of user input/prescription: in that case control unit 12 may control the blood pump 21 at a prefixed flow rate or at a flow rate calculated based on other parameters such as, for instance, other flow rates and constraints set by the medical operator (as apparent from the following description). In addition, or alternatively, the blood pump may also be controlled based on pressure; if the blood pump 21 is controlled based on the pressure signal detected upstream the blood pump then a pressure sensor 48 is present in the tract of bloodline upstream the blood pump 21: for instance the control unit 12 may be designed to drive the blood pump in a manner to keep the pressure detected by pressure sensor 48 within a prefixed range, or below a prefixed threshold.

Following the direction of blood circulation 200, in case the apparatus is also configured to remove CO₂ a gas exchanger 46 for removing CO₂ from circulating blood may be connected to the blood circuit. The gas exchanger 46 is in fluid communication with the blood circuit 17 to receive extracorporeal blood, allow CO₂ removal from blood and returning blood to the blood circuit at a downstream point. Figure 1 shows a gas exchanger 46 placed upstream the filtration unit 2; however, the gas exchanger may be alternatively positioned downstream the filtration unit on the blood return line 7. Notably, in case the CO₂ gas exchanger 46 is positioned downstream the filtration unit 2 on the blood return line 7, degassing in the blood withdrawal line increased its importance because air bubble in the filtration unit may cause blood clotting. As mentioned, blood circulation direction during normal use of the apparatus is indicated in figure 1 with an arrow 200 which also represent the blood flow rate Q_{b} direction during treatment. The gas exchanger 46 is connected in series with the filtration unit 2 and is placed downstream the injection point 50 where the infusion solution is delivered to extracorporeal blood. The gas exchanger 46 has a blood chamber and a gas chamber separated by a membrane permeable to gases, in particular CO₂; the gas exchanger comprise a gas inlet, which may be connected to a gas source, such as the medical gas supply system in a hospital to receive pressurized air or oxygen for example, and a gas outlet in fluid communication with the gas chamber to discharge exhausted gas having removed CO₂ from extracorporeal blood. The blood inlet and the blood outlet put the extracorporeal blood circuit 17 in fluid communication with the gas exchanger blood chamber. Clearly, the gas exchanger 46 may not be part of the dialysis apparatus should CO₂ removal be not necessary for the treatment.

Then, another pressure sensor 49 may be provided on the blood withdrawal line 6 for controlling the correct flow within the blood circuit: the pressure sensor 49 is interposed between the blood pump 21 and the filtration unit 2.

After passing through the primary chamber 3 of the filtration unit 2, where the suitable exchanges of substances, molecules and fluids occur by means of a semipermeable membrane, the treated blood enters the blood return line 7, first passing through the air separator 19, commonly known as "bubble trap", designed so as to ensure the detection and removal of air bubbles present in the blood. The treated blood getting out of the air separator 19, before being returned to the patient P passes through an air bubble sensor 55 verifying the absence of said dangerous formations within the treated blood that has to be re-introduced in the patient's blood circulation. Immediately downstream from the bubble sensor 55, the safety valve 20 (or venous clamp) is placed which, in case of alarm, may block the blood flow towards the patient. In particular, should the bubble sensor 55 detect the presence of air in the blood flow, the machine through safety valve 20 would be able to block immediately the passage of blood so as to avoid any consequence to the patient. A corresponding safety valve 27 (or arterial clamp) is present on the blood withdrawal line close the patient vascular access to fully isolate the patient from the extracorporeal blood circuit in case of need. Downstream from the safety valve 20, the treated blood is then carried back to the patient P undergoing therapy. The extracorporeal blood treatment apparatus of figure 1 is equipped with a dialysis fluid circuit 32, which is also provided with at least a dialysis supply line 8 leading into the filtration unit 2 and with an effluent (or dialysate) line 13 from the filtration unit. At least a primary fluid container, defining said dialysis liquid source 14, is designed to supply the supply line 8 of the dialysis fluid circuit 32 (generally the primary fluid container shall consist of one or more bags containing a suitable dialysis liquid). The supply line 8 includes actuator/s for conveying fluid such as at least a dialysis fluid pump 25 (in the embodiment of figure 1 a peristaltic pump) for controlling the flow rate Q_{dial} of dialysis liquid from the bag and for defining a direction 200 of dialysis fluid circulation. Downstream from the dialysis fluid pump 25 in the direction of circulation 200 there is a branching 56 splitting the dialysis supply line 8 up into an intake branch 57 and an infusion branch 58. In particular, the infusion branch 58 is connected to the blood return line 7 of the blood circuit 17. In other words, through said infusion branch 58 it is possible to obtain a post-infusion directly in the blood line 17 using the content of the primary fluid container. Conversely, the intake branch 57 conveys the fluid directly to the filtration unit 2 and in particular to the secondary chamber of said unit. The dialysis fluid circuit 32 is further equipped with a selector 59 for determining the percentages of fluid flow within the infusion branch 58 and the intake branch 57. Generally said selector 59, usually placed near the branching 56, may be positioned at least between a first operating condition in which it allows the passage of fluid in the intake branch 57 and blocks the passage in the infusion branch 58, and a second operating condition in which it allows the passage of fluid in the infusion branch 58 and blocks the passage in the intake branch 57. In other words, said selector 59 may consist of a valve element operating on the dialysis fluid circuit 32 by alternatively blocking the passage of fluid in either branch. Suitable selectors may be alternatively provided, which are able to establish a priori the amount of liquid that has to pass through both branches simultaneously. It will also be possible to vary the percentages of fluid in either branch as a function of time and of the pre-established therapies. The dialysis liquid through the intake branch 57 gets into the secondary chamber 4 of the filtration unit 2. In particular, the primary chamber 3 through which the blood flow passes is separated from the secondary chamber 4 through which the dialysis liquid passes through the semipermeable membrane 5 ensuring the suitable passage of the dangerous substances/molecules and of fluid from the blood towards the dialysis liquid mainly through convection and diffusion processes, and also ensuring through the same principles the passage of substances/molecules from the dialysis liquid towards the blood. The dialysis fluid then gets into the effluent line 13 and passes through a suitable effluent pressure sensor 60. An actuator is provided for conveying fluid, for instance a dialysate pump 26 controlling the flow rate Q_{eff} in the effluent line 13 within the fluid circuit 32. Also said pump will generally be a peristaltic pump. The fluid to be eliminated then passes through a blood detector 61 and is conveyed into a collection container or bag 62.

A further infusion line 51 for feeding fluid into the blood return line 7 of the blood circuit 17 may be provided. In particular, the infusion fluid is taken from at least an auxiliary container 64 and is sent directly to the blood return line 7 of the blood circuit 17 through actuator/s for conveying fluid, generally an infusion pump 65 (in the example a peristaltic pump) controlling its flow rate Qᵣₑₚ - total replacement flow rate. In particular, the infusion liquid may be introduced directly into the air separator 19. As can also be inferred, the infusion branch 58 of the dialysis fluid circuit 32 and the infusion line 63 are equipped with a common end length 66 letting fluid to enter into the blood circuit 17. Said intake end length 66 is placed downstream from the infusion pump 65 with respect to a direction of infusion and carries the fluid directly into the air separator 19. Further, referring to the diagram in figure 1, the infusion line 63 comprises at least a pre-infusion branch 67 connected to the blood withdrawal line 6 of the blood circuit 17. In further detail, downstream from the infusion pump 65 with respect to the direction of infusion, there is an infusion branching 68 splitting the infusion line 63 up into the pre-infusion branch 67 and post-infusion branch 69. The pre-infusion branch 67, in particular, carries the fluid taken from the bag 64 into the blood withdrawal line 6 of the blood circuit 17 downstream from the blood pump 21 and downstream the gas exchanger 46 with respect to the direction of blood circulation. Conversely, the post-infusion branch 69 is connected directly to the common end length 66. The infusion line 63 further comprises a selector 70 for determining the percentage of liquid flow to be sent to the post-infusion branch 69 and to the pre-infusion branch 67. The selector 70 placed near the branching 68 may be switched between at least a first operating condition in which it allows the passage of fluid in the pre-infusion branch 67 and blocks the passage in the post-infusion branch 69, and at least a second operating condition in which it allows the passage of fluid in the post-infusion branch 69 and blocks the passage in the pre-infusion branch 67. Obviously, as in the case of the selector 59 present on the dialysis fluid circuit 32, also the other selector 70 will be able to determine the percentage of fluid that has to pass in each of the two branches and to possibly vary it in time in accordance with the planned therapies. Moreover, the selector 59 and the other selector 70 will generally, though not necessarily, be of the same nature. Notably, the flow rate through the pre-infusion branch/line 67 may be determined by proper control of the infusion pump 65 and other selector 70; the control unit 12 may receive a pre-infusion ratio of replacement fluid flow PRE (a value between 0 and 1) and determine the pre-infusion flow rate Q_{rep.pre} based on the pre-infusion ratio PRE and on total replacement flow rate Qᵣₑₚ. In particular, Q_{rep.pre}=PRE·Qᵣₑₚ. Alternatively, post-infusion ratio may be used symmetrically, or a ratio between pre- and pos- infusion rate may be used as well (R=Q_{re.pre}/Q_{rep.post}).

The apparatus may be equipped with scales 71 for determining at least the weight of the primary fluid container 14 and/or of the auxiliary fluid container 64 and/or of the infusion substance source 10 and/or of the collection container 62. In particular, said scales 71 comprises weight sensors, for instance respective scales A, B, C, D and E (for example at least an independent sensor for each fluid bag associated to the machine). In particular, there will be at least four of said scales, each being independent from the other, and each one measuring the respective weight of a bag. It should then be pointed out that there is a control unit or CPU 12 active (at least) on the blood circuit 17 and in particular active on the pressure sensor 48 for reading pressure values, on the blood pump 21, on the gas exchanger 46, on the other pressure sensor 49, and on the device for detecting the presence of air bubbles 55 and on the respective safety valves 20, 27. The control unit 12 has also to control the dialysis fluid circuit 32 and, in particular, shall be input with the data detected by the scales A, B, C, D and (possibly) E and, concerning the weight of the bag 14, and shall act on the pump 25, on the selector 59, on the pressure sensor 60, then on the dialysate pump 26 and shall eventually receive the data detected by the scale A whose function is to determine the weight of the collection container 62. The control unit 12 shall also act on the infusion line 63 checking the weight of the auxiliary container 64 (checked by the scale C) and will be able to control both the infusion pump 54 65 and the other selector 70. The control unit 12 shall also act on the infusion line 51 detecting the weight of the infusion substance source 10 through the scale B and suitably controlling the infusion pump 54 according to the treatments to be carried out as below detailed and explained.

However, the apparatus of figure 1 may be alternatively (or additionally) provided with a systemic anticoagulation system, such as a syringe pump 9 for injecting heparin downstream the blood pump 21.

The control unit 12 is also connected to a memory and to user interface, for instance a graphic user interface, which receives operator's inputs and displays the apparatus outputs. For instance, the graphic user interface may include a touch screen, a display screen and/or hard keys for entering user's inputs or a combination thereof.

The control unit 12 is also connected to the blood pump 21 and configured to control the blood pump 21 to determine a blood flow rate in the blood withdrawal line 6. During an operating condition of the extracorporeal blood treatment, the control unit is configured to define a treatment condition wherein the blood pump 21 is set at a flow rate comprised between 50 ml/min and 600 ml/min, in particular between 100 ml/min and 350 ml/min, more in particular between 200 ml/min and 300 ml/min. Of course, the blood flow rate is set by the physician and may vary depending on various factors including the vascular access, the patient conditions, and the type of treatment.

In any case, during the patient treatment, the access negative pressure is experienced in the blood withdrawal line 6 upstream the blood pump tract 6p engaged by the blood pump 21.

On the other hand, the infusion pump 54 is operatively connected to the control unit 12 which is configured to selectively control the infusion pump 54 to promote the infusion fluid to flow within the infusion line 51 and to deliver the infusion fluid into the blood withdrawal line 6: the control unit 12 may be configured to control the infusion pump 54 to set the flow rate of the infusion fluid typically between 200 ml/h and 4000 ml/h, in particular between 500 ml/h and 2000 ml/h, more in particular at a flow rate substantially close to 1000 ml/h and lower than 1600 ml/h. Also in this regard, the type of treatment, the infusion bag content, and other conditions determine the set infusion rate, which is however normally set in the above discussed ranges.

The control unit may also be configured to deactivate the infusion pump 54 to arrest delivery of the infusion fluid.

According to an embodiment wherein the disposable set comprises the one-way valve 46, the control unit may be configured to define a first condition and a second condition.

In the first condition, the infusion pump 54 is active to generate the infusion fluid flow towards the blood withdrawal line 6 and the blood pump 21 is active to determine the blood flow in the blood circuit of the disposable set. In the first condition, the differential pressure across the one-way valve is higher than the preset opening pressure threshold of the one-way valve 46.This pressure difference causes the one-way valve 46 to switch to or to maintain the open position, allowing thereby the infusion fluid to be delivered into the blood withdrawal line 6. In other terms, the infusion pump 54 generates an over pressure which, combined with the low pressure caused by the blood pressure, determines the aperture of the one-way valve 46.

In the second condition, the infusion pump 54 is stopped to prevent infusion of the infusion fluid into the blood withdrawal line 6, while the blood pump is active: in the second condition the one-way valve 46 closes and prevents the infusion line 51 to be in fluid communication with the blood line. The access negative pressure is prevented to extend in the infusion line 51, thereby avoiding degassing of the infusion fluid.

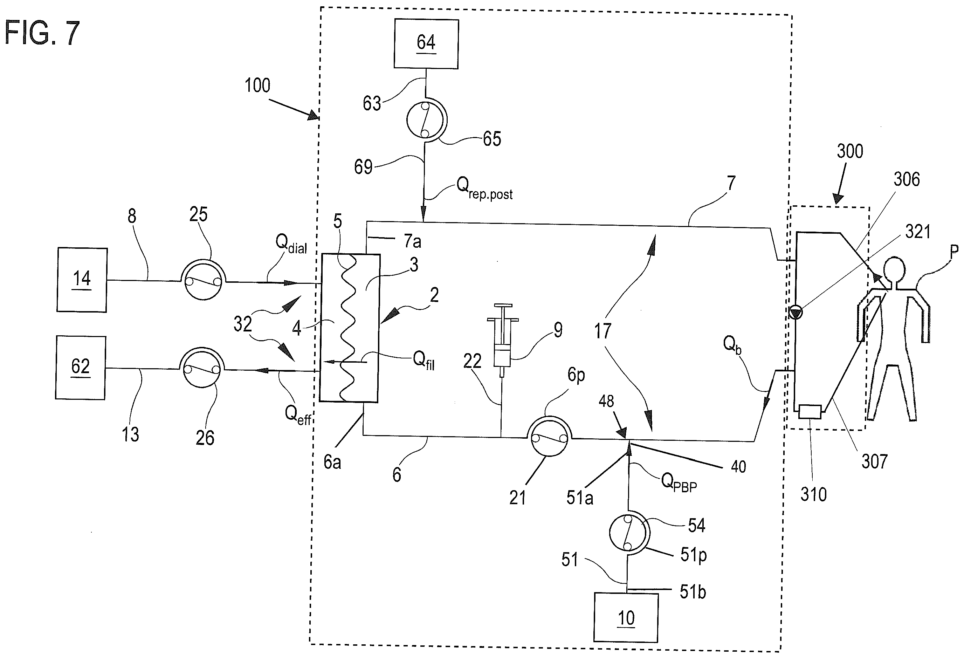

Figure 7 shows an embodiment wherein a disposable set 100 for extracorporeal blood treatment, for example a dialysis treatment, is connected to an Extra Corporeal Membrane Oxygenation ECMO circuit 300, which is in turn configured for connection to the patient. In other terms, the ECMO circuit 300 is fluidly interposed between the disposable set 100 and the patient's vascular accesses.

The ECMO circuit is only very schematically represented in order to show a further extracorporeal blood flow circulation. Clearly, the ECMO circuit includes all necessary lines and components for proper working. No further details are provided about a detailed embodiment of the ECMO circuit: anyhow, the skilled person knows the key features of an ECMO circuit and the main elements/devices associated without further explanation. Notably, the ECMO circuit is not per se part of the present invention, while it is an extra circuit where the disposable set 100 of the present invention may be coupled to, instead of directly connecting the disposable set 100 to the patient. Indeed, when blood oxygenation and a dialysis treatment are proper, a layout as the one proposed in figure 7 may be implemented in order to avoid multiple blood accesses to the patient.

Clearly any suitable connection of the extracorporeal blood circuit 100 to the ECMO apparatus may be used. The blood withdrawal line 6 and/or the blood return line 7 may be connected to respective blood lines of the ECMO apparatus wherein positive or negative pressure occurs. Two examples of possible connection are below briefly described. However, what is relevant here is that: (i) the extracorporeal blood circuit 100 may not be directly connected to the patient access (ii) any one of the blood withdrawal line 6 and the blood return line 7 may experience a negative pressure regimen and therefore the corresponding pressure damper may be usefully applied at the junction point of any infusion line injecting into the blood circuit where there is a negative pressure in the blood at the junction point.

According to the embodiment of figure 7, the blood withdrawal line 6 of the disposable set 100 is connected to the ECMO circuit 300 downstream the blood pump 321 of the ECMO circuit, while the blood return line 7 of the disposable set 100 is connected to the ECMO circuit 300 upstream the blood pump 321 of the ECMO circuit. In this case, the blood withdrawal line 6 of the disposable line experience a positive pressure: thus, the pressure at the cross between the blood withdrawal line 6 and the infusion line 54 in the disposable set 100 prevents infusion fluid degassing. However, the blood return line 7 may experience a negative pressure regimen. In this case, the use of a pressure damper arranged proximate or at the first end of the post infusion line 69 may be used to prevent degassing in the post infusion line itself. The pressure damper is configured to prevent, or reduce an amount of, the negative pressure to extend in the post infusion line 69 upstream the pressure damper. The same embodiments of pressure dampers already discussed in the previous sections may be used here.

In a second ECMO embodiment not shown in the attached figured, the blood withdrawal line 6 of the disposable set 100 may be connected to the ECMO circuit 300 upstream the blood pump 321 of the ECMO circuit, while the blood return line 7 of the disposable set 100 may be connected to the ECMO circuit 300 downstream the blood pump 321 of the ECMO circuit. In this case, the pressure at the fluid access 48 between the blood withdrawal line 6 and the infusion line 54 in the disposable set 100 may be negative. Thus, the infusion fluid in the infusion line 51 may be subjected to a low pressure, which may cause fluid degassing. Thus, a disposable set according to the present invention may allow avoiding fluid degassing in the fluid line 51 when connected to an ECMO circuit, when the blood line having the infusion line 51 is connected to the ECMO circuit upstream the blood pump 321 of the ECMO circuit.

## Claims

1. Disposable set for an extracorporeal blood treatment apparatus (1), the disposable set (100) comprising:
- a filtration unit (2);
- a blood circuit (17) comprising:
∘ a blood withdrawal line (6) extending between a first end (6a) connected to the filtration unit (2) and a second end (6b),
∘ a blood return line (7) extending between a first end (7a) connected to the filtration unit (2) and a second end (7b);
the blood withdrawal line (6) including a blood pump tract (6p) configured to be engaged by a blood pump (21) of the extracorporeal blood treatment apparatus (1) which is configured to determine a blood flow, at least during an operating condition, an access negative pressure is experienced upstream said blood pump tract (6p), said blood flow in the blood circuit (17) being in a direction from the blood withdrawal line (6) towards the filtration unit (2) and from the filtration unit (2) through the blood return line (7);
- an infusion line (51) extending between a first end (51a) connected to the blood circuit (17) at a fluid access, the first end (51a) being connected to the blood withdrawal line (6) upstream the blood pump tract (6p), and a second end (51b) for connection to an infusion substance source (10), wherein the blood pump tract (6p) is interposed between the second end (7b) of the blood return line (7), in particular the filtration unit (2), and the first end of the infusion line (51);
- wherein the disposable set further comprises a pressure damper (40) arranged proximate or at the first end of the infusion line (51), the pressure damper (40) comprises a one-way valve (46) configured to allow fluid passage only in an infusion direction directed from the infusion line (51) towards the blood withdrawal line (6), and is configured to move between an open position, in which the fluid passage is allowed in said infusion direction, and a closed position, in which fluid passage is prevented in both directions,
**characterized in that** the pressure damper (40) is configured to prevent the access negative pressure to extend in the infusion line (51) upstream the pressure damper (40),
the one-way valve (46) being preset at an opening pressure threshold corresponding to a differential pressure value higher than 160 mmHg to switch between the closed and the open position and vice versa, so that:
∘ if a differential pressure between an upstream section and a downstream section of the one-way valve (46) is equal or higher than said opening pressure threshold, the one-way valve (46) is configured to switch to or to maintain the open position,
and
∘ if said differential pressure is lower than said opening pressure threshold, the one-way valve (46) is configured to switch to or to maintain the closed position.

2. Disposable set according to claim 1, wherein the preset opening pressure threshold of the one-way valve (46) corresponds to a differential pressure value comprised between 160 and 500 mmHg, in particular between 190 and 450 mmHg, more in particular between 200 and 400 mmHg, more in detail between 240 and 350 mmHg.

3. Disposable set according to any one of the preceding claims 1 and 2, wherein the preset opening pressure threshold of the one-way valve (46) is set about a maximum negative pressure allowed at the fluid access during standard working condition of the extracorporeal blood treatment apparatus (1), in particular within +/-100 mmHg, and more particularly within +/-50 mmHg the maximum negative pressure allowed at the fluid access.

4. Disposable set according to any one of the preceding claims 1 to 3, wherein said one-way valve (46) is arranged on the infusion line (51) at a distance from the first end of the infusion line (51) no longer than 6 cm, optionally no longer that 3 cm, in particular comprised between 0.1 cm and 6 cm, more in particular between 0.2 and 3 cm.

5. Disposable set according to any one of the preceding claims, the disposable set comprising an infusion connector (48) including:
- a connector body defining an internal volume;
- a blood inlet and a blood outlet fluidly communicating each other and with said internal volume, the blood inlet being connected to the blood withdrawal line (6) facing the second end of the blood withdrawal line, and the blood outlet being connected to the blood withdrawal line (6) facing the blood pump tract (6p) of the blood withdrawal line (6);
- an infusion inlet fluidly communicating with said internal volume and connected to the first end of the infusion line (51),
wherein the infusion connector is a three-way connector, in particular having two inlets and one outlet,
and wherein the pressure damper (40) is arranged within the infusion inlet of the infusion connector.

6. Disposable set according to any one of the preceding claims, wherein the infusion line (51) comprises a respective infusion pump tract (51p) interposed between the first end and the second end of the infusion line (51), the infusion pump tract of the infusion line (51) being configured to be engaged by an infusion peristaltic pump configured to determine, at least during an operating condition, a positive pressure downstream the infusion pump tract to allow the infusion fluid to flow in a direction towards the first end of the infusion line (51) and towards the blood withdrawal line (6),
wherein the pressure damper (40) is arranged on the infusion line (51) between the infusion pump tract (51p) and the blood withdrawal line (6).

7. Disposable set according to any one of the preceding claims, wherein the one-way valve (46) comprises an internal diaphragm (46a) movable between the open position and the closed position, the internal diaphragm (46a) being preloaded in the closed position, said preload defining the preset opening pressure threshold, in particular the internal diaphragm being a flexible membrane made of a material between PVC, silicone, rubber.

8. Disposable set according to the preceding claim, comprising a Luer lock connector (47) housing said one-way valve (46), in particular wherein said internal diaphragm (46a) is within an internal fluid passage of the Luer lock connector.

9. Disposable set according to any one of the preceding claims, wherein the blood withdrawal line (6) of the blood circuit has a fluid passage section comprised between 3 mm² and 20 mm² or a diameter comprised between 2 mm and 5 mm and the infusion line (51) has a fluid passage section comprised between a diameter comprised between 1 mm and 4 mm.

10. Disposable set according to any one of the preceding claims, further comprising a fluid circuit comprising:
- an effluent fluid line (13) extending between a first end connected to an outlet of a secondary chamber (4) of the filtration unit (2), and a second end for connection to a drain or a collection container (62), the effluent fluid line (13) comprising a respective pump tract configured to be engaged to a dialysate pump (26),
- a dialysis liquid supply line (8) extended between a first end connected to an inlet of a secondary chamber of the filtration unit (2), and a second end for connection to a dialysis fluid source, the dialysis liquid supply line (8) comprising a respective supply pump tract configured to be engaged to a dialysis fluid pump (25).

11. Extracorporeal blood treatment apparatus comprising:
- the disposable set according to any one of the preceding claims 1 to 10,
- a blood pump cooperating with the blood pump tract (6p) of the blood withdrawal line (6),
- an infusion pump (54) cooperating with an infusion pump tract (51p) of the infusion line (51), said infusion pump (54) being arranged between the second end of the infusion line (51) and the pressure damper (40), in particular upstream the pressure damper (40) and downstream the infusion substance source (10),
- a control unit (12) operatively connected to the blood pump (21) and the infusion pump (54), the control unit (12) being configured to control the blood pump (21) and the infusion pump (54) at a corresponding blood flow rate and an infusion flow rate,
the control unit being configured to define a treatment condition wherein:
- the blood flow rate is comprised between 50 ml/min and 600 ml/min, in particular between 50 ml/min and 350 ml/min, more in particular between 100 ml/min and 300 ml/min, and
- the infusion flow rate is comprised between 200 ml/h and 4000 ml/h, in particular higher than 500 ml/h and lower than 2000 ml/h.

12. Extracorporeal blood treatment apparatus according to the preceding claim,
wherein the control unit is configured to define:
- a first condition wherein the infusion pump (54) is active to determine a fluid flow, an infusion positive pressure is present downstream the infusion pump, and the blood pump is active to determine a blood flow, an access negative pressure is present upstream the blood pump,
wherein a pressure difference between said infusion positive pressure and the access negative pressure is higher than the preset opening pressure threshold of the one-way valve (46) to allow the infusion fluid to enter the blood withdrawal line (6), and
- a second condition wherein the infusion pump (54) is stopped to prevent infusion of the infusion fluid into the blood withdrawal line (6), and the blood pump (21) is active to determine blood flow in the blood circuit, in said second condition the one-way valve (46) is closed as a difference between a pressure in the infusion line (51) and the access negative pressure is lower than the preset opening pressure threshold of the one-way valve (46) preventing the access negative pressure to extend in the infusion line (51).

13. Extracorporeal blood treatment apparatus according to claims 11 or 12, wherein the extracorporeal blood treatment apparatus 1 is designed for continuous renal replacement therapies (CRRT).

## Patentansprüche

1. Einwegset für eine extrakorporale Blutbehandlungsvorrichtung (1), wobei das Einwegset (100) umfasst:
- eine Filtrationseinheit (2);
- einen Blutkreislauf (17), der umfasst:
∘ eine Blutentnahmeleitung (6), die sich zwischen einem ersten Ende (6a), das mit der Filtrationseinheit (2) verbunden ist, und einem zweiten Ende (6b) erstreckt,
∘ eine Blutrückführleitung (7), die sich zwischen einem ersten Ende (7a), das mit der Filtrationseinheit (2) verbunden ist, und einem zweiten Ende (7b) erstreckt;
wobei die Blutentnahmeleitung (6) einen Blutpumpentrakt (6p) aufweist, der dazu ausgelegt ist, von einer Blutpumpe (21) der extrakorporalen Blutbehandlungsvorrichtung (1) in Eingriff genommen zu werden, die dazu ausgelegt ist, einen Blutfluss zu bestimmen, zumindest wird während eines Betriebszustands stromaufwärts des Blutpumpentrakts (6p) ein Zugangsunterdruck erfahren, wobei der Blutfluss im Blutkreislauf (17) in einer Richtung von der Blutentnahmeleitung (6) zur Filtrationseinheit (2) und von der Filtrationseinheit (2) durch die Blutrückführleitung (7) verläuft;
- eine Infusionsleitung (51), die sich zwischen einem ersten Ende (51a), das mit dem Blutkreislauf (17) an einem Fluidzugang verbunden ist, wobei das erste Ende (51a) mit der Blutentnahmeleitung (6) stromaufwärts des Blutpumpentrakts (6p) verbunden ist, und einem zweiten Ende (51b) zur Verbindung mit einer Infusionssubstanzquelle (10) erstreckt, wobei der Blutpumpentrakt (6p) zwischen dem zweiten Ende (7b) der Blutrückführleitung (7), insbesondere der Filtrationseinheit (2), und dem ersten Ende der Infusionsleitung (51) angeordnet ist;
- wobei das Einwegset ferner einen Druckdämpfer (40) umfasst, der nahe oder an dem ersten Ende der Infusionsleitung (51) angeordnet ist, wobei der Druckdämpfer (40) ein Einwegventil (46) umfasst, das dazu ausgelegt ist, einen Fluiddurchgang nur in einer Infusionsrichtung zu ermöglichen, die von der Infusionsleitung (51) zu der Blutentnahmeleitung (6) gerichtet ist, und dazu ausgelegt ist, sich zwischen einer offenen Position, in der der Fluiddurchgang in der Infusionsrichtung ermöglicht wird, und einer geschlossenen Position, in der ein Fluiddurchgang in beiden Richtungen verhindert wird, zu bewegen,
**dadurch gekennzeichnet, dass** der Druckdämpfer (40) dazu ausgelegt ist, zu verhindern, dass sich der Zugangsunterdruck in der Infusionsleitung (51) stromaufwärts des Druckdämpfers (40) erstreckt,
wobei das Einwegventil (46) auf eine Öffnungsdruckschwelle voreingestellt ist, die einem Differenzdruckwert von mehr als 160 mmHg entspricht, um zwischen der geschlossenen und der offenen Position und umgekehrt zu wechseln, so dass:
∘ wenn ein Differenzdruck zwischen einem stromaufwärtigen Abschnitt und einem stromabwärtigen Abschnitt des Einwegventils (46) gleich oder höher ist als die Öffnungsdruckschwelle, das Einwegventil (46) dazu ausgelegt ist, in die offene Position zu wechseln oder die offene Position beizubehalten,
und
∘ wenn der Differenzdruck niedriger ist als die Öffnungsdruckschwelle, das Einwegventil (46) dazu ausgelegt ist, in die geschlossene Position zu wechseln oder die geschlossene Position beizubehalten.

2. Einwegset nach Anspruch 1, wobei die voreingestellte Öffnungsdruckschwelle des Einwegventils (46) einem Differenzdruckwert zwischen 160 und 500 mmHg, insbesondere zwischen 190 und 450 mmHg, insbesondere zwischen 200 und 400 mmHg, genauer zwischen 240 und 350 mmHg entspricht.

3. Einwegset nach einem der vorhergehenden Ansprüche 1 und 2, wobei die voreingestellte Öffnungsdruckschwelle des Einwegventils (46) auf einen maximalen Unterdruck eingestellt ist, der während eines Standardarbeitszustands der extrakorporalen Blutbehandlungsvorrichtung (1) am Fluidzugang zugelassen ist, insbesondere innerhalb +/-100 mmHg und insbesondere innerhalb +/-50 mmHg des maximalen Unterdrucks, der am Fluidzugang zugelassen ist.

4. Einwegset nach einem der vorhergehenden Ansprüche 1 bis 3, wobei das Einwegventil (46) an der Infusionsleitung (51) in einem Abstand vom ersten Ende der Infusionsleitung (51) von nicht mehr als 6 cm, optional nicht mehr als 3 cm, insbesondere zwischen 0,1 cm und 6 cm, insbesondere zwischen 0,2 und 3 cm, angeordnet ist.

5. Einwegset nach einem der vorhergehenden Ansprüche, wobei das Einwegset einen Infusionsverbinder (48) umfasst, der aufweist:
- einen Verbinderkörper, der ein Innenvolumen definiert;
- einen Bluteinlass und einen Blutauslass, die miteinander und mit dem Innenvolumen in Fluidverbindung stehen, wobei der Bluteinlass mit der Blutentnahmeleitung (6) verbunden ist und dem zweiten Ende der Blutentnahmeleitung zugewandt ist, und wobei der Blutauslass mit der Blutentnahmeleitung (6) verbunden ist und dem Blutpumpentrakt (6p) der Blutentnahmeleitung (6) zugewandt ist;
- einen Infusionseinlass, der mit dem Innenvolumen in Fluidverbindung steht und mit dem ersten Ende der Infusionsleitung (51) verbunden ist,
wobei der Infusionsverbinder ein Dreiwegeverbinder ist, insbesondere mit zwei Einlässen und einem Auslass,
und wobei der Druckdämpfer (40) innerhalb des Infusionseinlasses des Infusionsverbinders angeordnet ist.

6. Einwegset nach einem der vorhergehenden Ansprüche, wobei die Infusionsleitung (51) einen jeweiligen Infusionspumpentrakt (51p) umfasst, der zwischen dem ersten Ende und dem zweiten Ende der Infusionsleitung (51) angeordnet ist, wobei der Infusionspumpentrakt der Infusionsleitung (51) dazu ausgelegt ist, von einer Infusionsperistaltikpumpe in Eingriff genommen zu werden, die dazu ausgelegt ist, zumindest während eines Betriebszustands einen Überdruck stromabwärts des Infusionspumpentrakts zu bestimmen, um zu ermöglichen, dass die Infusionsflüssigkeit in eine Richtung des ersten Endes der Infusionsleitung (51) und in Richtung der Blutentnahmeleitung (6) fließt,
wobei der Druckdämpfer (40) an der Infusionsleitung (51) zwischen dem Infusionspumpentrakt (51p) und der Blutentnahmeleitung (6) angeordnet ist.

7. Einwegset nach einem der vorhergehenden Ansprüche, wobei das Einwegventil (46) eine zwischen der offenen Position und der geschlossenen Position bewegliche innere Membran (46a) umfasst, wobei die innere Membran (46a) in der geschlossenen Position vorgespannt ist, wobei die Vorspannung die voreingestellte Öffnungsdruckschwelle definiert, wobei insbesondere die innere Membran eine flexible Membran ist, die aus einem Material zwischen PVC, Silikon, Gummi hergestellt ist.

8. Einwegset nach dem vorhergehenden Anspruch, umfassend einen Luer-Lock-Verbinder (47), der das Einwegventil (46) aufnimmt, wobei sich insbesondere die innere Membran (46a) innerhalb eines inneren Fluiddurchgangs des Luer-Lock-Verbinders befindet.

9. Einwegset nach einem der vorhergehenden Ansprüche, wobei die Blutentnahmeleitung (6) des Blutkreislaufs einen Fluiddurchgangsabschnitt aufweist, der zwischen 3 mm² und 20 mm² liegt, oder einen Durchmesser, der zwischen 2 mm und 5 mm liegt, und die Infusionsleitung (51) einen Fluiddurchgangsabschnitt aufweist, der zwischen 1 mm und 4 mm liegt.

10. Einwegset nach einem der vorhergehenden Ansprüche, ferner umfassend einen Fluidkreislauf, umfassend:
- eine Abflussfluidleitung (13), die sich zwischen einem ersten Ende, das mit einem Auslass einer Sekundärkammer (4) der Filtrationseinheit (2) verbunden ist, und einem zweiten Ende zur Verbindung mit einem Abfluss oder einem Sammelbehälter (62) erstreckt, wobei die Abflussfluidleitung (13) einen jeweiligen Pumpentrakt umfasst, der dazu ausgelegt ist, von einer Dialysatpumpe (26) in Eingriff genommen zu werden,
- eine Dialyseflüssigkeitszuleitung (8), die sich zwischen einem ersten Ende, das mit einem Einlass einer Sekundärkammer der Filtrationseinheit (2) verbunden ist, und einem zweiten Ende zur Verbindung mit einer Dialyseflüssigkeitsquelle erstreckt, wobei die Dialyseflüssigkeitszuleitung (8) einen jeweiligen Versorgungspumpentrakt umfasst, der dazu ausgelegt ist, von einer Dialyseflüssigkeitspumpe (25) in Eingriff genommen zu werden.

11. Extrakorporale Blutbehandlungsvorrichtung, umfassend:
- das Einwegset nach einem der vorhergehenden Ansprüche 1 bis 10,
- eine Blutpumpe, die mit dem Blutpumpentrakt (6p) der Blutentnahmeleitung (6) zusammenwirkt;
- eine Infusionspumpe (54), die mit einem Infusionspumpentrakt (51p) der Infusionsleitung (51) zusammenwirkt, wobei die Infusionspumpe (54) zwischen dem zweiten Ende der Infusionsleitung (51) und dem Druckdämpfer (40), insbesondere stromaufwärts des Druckdämpfers (40) und stromabwärts der Infusionssubstanzquelle (10), angeordnet ist;
- eine Steuereinheit (12), die funktional mit der Blutpumpe (21) und der Infusionspumpe (54) verbunden ist, wobei die Steuereinheit (12) dazu ausgelegt ist, die Blutpumpe (21) und die Infusionspumpe (54) mit einer entsprechenden Blutflussrate und einer Infusionsflussrate zu steuern,
wobei die Steuereinheit dazu ausgelegt ist, einen Behandlungszustand zu definieren, wobei:
- die Blutflussrate zwischen 50 ml/min und 600 ml/min, insbesondere zwischen 50 ml/min und 350 ml/min, insbesondere zwischen 100 ml/min und 300 ml/min liegt, und
- die Infusionsflussrate zwischen 200 ml/h und 4000 ml/h, insbesondere über 500 ml/h und unter 2000 ml/h liegt.

12. Extrakorporale Blutbehandlungseinrichtung nach dem vorhergehenden Anspruch, wobei die Steuereinheit dazu ausgelegt ist, zu definieren:
- einen ersten Zustand, in dem die Infusionspumpe (54) aktiv ist, um einen Fluidstrom zu bestimmen, und ein Infusionsüberdruck stromabwärts der Infusionspumpe vorhanden ist, und die Blutpumpe aktiv ist, um einen Blutstrom zu bestimmen, und ein Zugangsunterdruck stromaufwärts der Blutpumpe vorhanden ist,
wobei eine Druckdifferenz zwischen dem Infusionsüberdruck und dem Zugangsunterdruck höher ist als die voreingestellte Öffnungsdruckschwelle des Einwegventils (46), um zu ermöglichen, dass das Infusionsfluid in die Blutentnahmeleitung (6) eintritt; und
- einen zweiten Zustand, in dem die Infusionspumpe (54) gestoppt wird, um eine Infusion der Infusionsflüssigkeit in die Blutentnahmeleitung (6) zu verhindern, und die Blutpumpe (21) aktiv ist, um den Blutfluss im Blutkreislauf zu bestimmen, wobei in dem zweiten Zustand das Einwegventil (46) geschlossen ist, da eine Differenz zwischen einem Druck in der Infusionsleitung (51) und dem Zugangsunterdruck niedriger ist als die vorgegebene Öffnungsdruckschwelle des Einwegventils (46), wodurch verhindert wird, dass sich der Zugangsunterdruck in der Infusionsleitung (51) erstreckt.

13. Extrakorporale Blutbehandlungsvorrichtung nach Anspruch 11 oder 12, wobei die extrakorporale Blutbehandlungsvorrichtung 1 für kontinuierliche Nierenersatztherapien (CRRT) entwickelt ist.

## Revendications

1. Ensemble jetable pour un appareil de traitement extracorporel du sang (1), l'ensemble jetable (100) comprenant :
- une unité de filtration (2) ;
- un circuit sanguin (17) comprenant :
∘ une ligne de prélèvement sanguin (6) s'étendant entre une première extrémité (6a) reliée à l'unité de filtration (2) et une seconde extrémité (6b),
∘ une ligne de retour sanguin (7) s'étendant entre une première extrémité (7a) reliée à l'unité de filtration (2) et une seconde extrémité (7b) ;
la ligne de prélèvement sanguin (6) comportant un tronçon de pompe à sang (6p) configuré pour être en prise avec une pompe à sang (21) de l'appareil de traitement extracorporel du sang (1) qui est configurée pour déterminer un flux sanguin, au moins pendant une condition de fonctionnement, une dépression d'accès est subie en amont dudit tronçon de pompe à sang (6p), ledit flux sanguin dans le circuit sanguin (17) étant dans un sens allant de la ligne de prélèvement sanguin (6) vers l'unité de filtration (2) et de l'unité de filtration (2) à travers la ligne de retour sanguin (7) ;
- une ligne de perfusion (51) s'étendant entre une première extrémité (51a) reliée au circuit sanguin (17) au niveau d'un accès fluidique, la première extrémité (51a) étant reliée à la ligne de prélèvement sanguin (6) en amont du tronçon de pompe à sang (6p), et une seconde extrémité (51b) de liaison à une source (10) de substance pour perfusion, dans lequel le tronçon (6p) est interposé entre la seconde extrémité (7b) de la ligne de retour sanguin (7), en particulier l'unité de filtration (2), et la première extrémité de la ligne de perfusion (51) ;
- dans lequel l'ensemble jetable comprend en outre un amortisseur de pression (40) agencé à proximité ou au niveau de la première extrémité de la ligne de perfusion (51), l'amortisseur de pression (40) comprend une vanne unidirectionnelle (46) configurée pour ne laisser passer de fluide que dans un sens de perfusion dirigé de la ligne de perfusion (51) vers la ligne de prélèvement sanguin (6), et est configuré pour se déplacer entre une position ouverte, dans laquelle le passage de fluide est autorisé dans ledit sens de perfusion, et une position fermée, dans laquelle le passage de fluide est empêché dans les deux sens,
**caractérisé en ce que** l'amortisseur de pression (40) est configuré pour empêcher la dépression d'accès de s'étendre dans la ligne de perfusion (51) en amont de l'amortisseur de pression (40),
la vanne unidirectionnelle (46) étant préréglée à un seuil de pression d'ouverture correspondant à une valeur de pression différentielle supérieure à 160 mmHg pour basculer entre la position fermée et la position ouverte et inversement, de sorte que :
∘ si une pression différentielle entre une section amont et une section aval de la vanne unidirectionnelle (46) est égale ou supérieure audit seuil de pression d'ouverture, la vanne unidirectionnelle (46) est configurée pour basculer vers la position ouverte ou conserver celle-ci,
et
∘ si ladite pression différentielle est inférieure audit seuil de pression d'ouverture, la vanne unidirectionnelle (46) est configurée pour basculer vers la position fermée ou conserver celle-ci.

2. Ensemble jetable selon la revendication 1, dans lequel le seuil de pression d'ouverture prédéfini de la vanne unidirectionnelle (46) correspond à une valeur de pression différentielle comprise entre 160 et 500 mmHg, en particulier entre 190 et 450 mmHg, plus particulièrement entre 200 et 400 mmHg, plus précisément entre 240 et 350 mmHg.

3. Ensemble jetable selon l'une quelconque des revendications 1 et 2 précédentes, dans lequel le seuil de pression d'ouverture prédéfini de la vanne unidirectionnelle (46) est réglé autour d'une dépression maximale autorisée au niveau de l'accès fluidique pendant la condition de fonctionnement normal de l'appareil extracorporel de traitement du sang (1), en particulier à +/-100 mmHg, et plus particulièrement à +/-50 mmHg de la dépression maximale autorisée au niveau de l'accès fluidique.

4. Ensemble jetable selon l'une quelconque des revendications 1 à 3 précédentes, dans lequel ladite vanne unidirectionnelle (46) est agencée sur la ligne de perfusion (51) à une distance de la première extrémité de la ligne de perfusion (51) qui n'est pas supérieure à 6 cm, éventuellement pas supérieure à 3 cm, en particulier comprise entre 0,1 cm et 6 cm, plus particulièrement entre 0,2 et 3 cm.

5. Ensemble jetable selon l'une quelconque des revendications précédentes, l'ensemble jetable comprenant un raccord de perfusion (48) comportant :
- un corps de raccord définissant un volume interne ;
- une entrée de sang et une sortie de sang communiquant fluidiquement entre elles et avec ledit volume interne, l'entrée de sang étant reliée à la ligne de prélèvement sanguin (6) en regard de la seconde extrémité de la ligne de prélèvement sanguin, et la sortie de sang étant reliée à la ligne de prélèvement sanguin (6) en regard du tronçon de pompe à sang (6p) de la ligne de prélèvement sanguin (6),
- une entrée de perfusion communiquant fluidiquement avec ledit volume interne et reliée à la première extrémité de la ligne de perfusion (51),
dans lequel le raccord de perfusion est un raccord à trois voies, en particulier ayant deux entrées et une sortie,
et dans lequel l'amortisseur de pression (40) est agencé à l'intérieur de l'entrée de perfusion du raccord de perfusion.

6. Ensemble jetable selon l'une quelconque des revendications précédentes, dans lequel la ligne de perfusion (51) comprend un tronçon de pompe de perfusion (51p) respectif interposé entre la première extrémité et la seconde extrémité de la ligne de perfusion (51), le tronçon de pompe de perfusion de la ligne de perfusion (51) étant configuré pour être en prise avec une pompe péristaltique de perfusion configurée pour déterminer, au moins pendant une condition de fonctionnement, une pression positive en aval du tronçon de pompe de perfusion pour permettre au fluide de perfusion de s'écouler **en direction de** la première extrémité de la ligne de perfusion (51) et vers la ligne de prélèvement sanguin (6),
dans lequel l'amortisseur de pression (40) est disposé sur la ligne de perfusion (51) entre le tronçon de pompe de perfusion (51p) et la ligne de prélèvement sanguin (6).

7. Ensemble jetable selon l'une quelconque des revendications précédentes, dans lequel la vanne unidirectionnelle (46) comprend une membrane interne (46a) mobile entre la position ouverte et la position fermée, la membrane interne (46a) étant précontrainte en position fermée, ladite précontrainte définissant le seuil de pression d'ouverture prédéfini, en particulier la membrane interne étant une membrane souple réalisée dans un matériau entre PVC, silicone, caoutchouc.

8. Ensemble jetable selon la revendication précédente, comprenant un raccord Luer Lock (47) logeant ladite vanne unidirectionnelle (46), en particulier dans lequel ladite membrane interne (46a) se trouve à l'intérieur d'un passage de fluide interne du raccord Luer Lock.

9. Ensemble jetable selon l'une quelconque des revendications précédentes, dans lequel la ligne de prélèvement sanguin (6) du circuit sanguin a une section de passage de fluide comprise entre 3 mm² et 20 mm² ou un diamètre compris entre 2 mm et 5 mm et la ligne de perfusion (51) a une section de passage de fluide comprise entre un diamètre compris entre 1 mm et 4 mm.

10. Ensemble jetable selon l'une quelconque des revendications précédentes, comprenant en outre un circuit fluidique comprenant :
- une ligne de fluide effluent (13) s'étendant entre une première extrémité reliée à une sortie d'une chambre secondaire (4) de l'unité de filtration (2), et une seconde extrémité pour un raccordement à une évacuation ou un récipient de collecte (62), la ligne de fluide effluent (13) comprenant un tronçon de pompe respective configuré pour être en prise avec une pompe de dialysat (26),
- une ligne d'alimentation en liquide de dialyse (8) s'étendant entre une première extrémité reliée à une entrée d'une chambre secondaire de l'unité de filtration (2), et une seconde extrémité pour un raccordement à une source de fluide de dialyse, la ligne d'alimentation en liquide de dialyse (8) comprenant un tronçon de pompe d'alimentation respective configuré pour être en prise avec une pompe de fluide de dialyse (25).

11. Appareil de traitement extracorporel du sang comprenant :
- l'ensemble jetable selon l'une quelconque des revendications 1 à 10 précédentes,
- une pompe à sang coopérant avec le tronçon de pompe à sang (6p) de la ligne de prélèvement sanguin (6),
- une pompe de perfusion (54) coopérant avec un tronçon de pompe de perfusion (51p) de la ligne de perfusion (51), ladite pompe de perfusion (54) étant agencée entre la seconde extrémité de la ligne de perfusion (51) et l'amortisseur de pression (40), en particulier en amont de l'amortisseur de pression (40) et en aval de la source de substance de perfusion (10),
- une unité de commande (12) reliée de manière fonctionnelle à la pompe à sang (21) et à la pompe de perfusion (54), l'unité de commande (12) étant configurée pour commander la pompe à sang (21) et la pompe de perfusion (54) à un débit sanguin et un débit de perfusion correspondants,
l'unité de commande étant configurée pour définir une condition de traitement dans laquelle :
- le débit sanguin est compris entre 50 ml/min et 600 ml/min, en particulier entre 50 ml/min et 350 ml/min, plus particulièrement entre 100 ml/min et 300 ml/min, et
- le débit de perfusion est compris entre 200 ml/h et 4 000 ml/h, en particulier supérieur à 500 ml/h et inférieur à 2 000 ml/h.

12. Appareil de traitement extracorporel du sang selon la revendication précédente, dans lequel l'unité de commande est configurée pour définir :
- une première condition dans laquelle la pompe de perfusion (54) est active pour déterminer un écoulement de fluide, une pression positive de perfusion existe en aval de la pompe de perfusion et la pompe à sang est active pour déterminer un flux sanguin, une dépression d'accès existe en amont de la pompe à sang,
dans lequel une différence de pression entre ladite pression positive de perfusion et la dépression d'accès est supérieure au seuil de pression d'ouverture prédéfini de la vanne unidirectionnelle (46) pour permettre au fluide de perfusion d'entrer dans la ligne de prélèvement sanguin (6) ; et
- une seconde condition dans laquelle la pompe de perfusion (54) est arrêtée pour empêcher la perfusion du fluide de perfusion dans la ligne de prélèvement sanguin (6), et la pompe à sang (21) est active pour déterminer le flux sanguin dans le circuit sanguin, dans ladite seconde condition, la vanne unidirectionnelle (46) est fermée, car la différence entre une pression dans la ligne de perfusion (51) et la dépression d'accès est inférieure au seuil de pression d'ouverture prédéfini de la vanne unidirectionnelle (46), empêchant ainsi la dépression d'accès de s'étendre dans la ligne de perfusion (51).

13. Appareil extracorporel de traitement du sang selon la revendication 11 ou 12, l'appareil extracorporel de traitement du sang 1 étant conçu pour des thérapies de remplacement rénal en continu (CRRT).
